# EUROPÄISCHE PATENTSCHRIFT

(11) **EP 4 486 271 B1**
(45) Veröffentlichungstag und Bekanntmachung des Hinweises auf die Patenterteilung: **29.10.2025**
(21) Anmeldenummer: 23709390.1
(22) Anmeldetag: 03.03.2023
(51) Int. Cl.: A61F 13/00, A61L 2/14, H05H 1/24

(54) **WUNDAUFLAGE**
WOUND DRESSING
PANSEMENT

(30) Priorität: 04.03.2022 DE 102022105186
(43) Veröffentlichungstag der Anmeldung: 08.01.2025
(73) Patentinhaber: Cinogy GmbH, 37115 Duderstadt (DE)
(72) Erfinder: WANDKE, Dirk, 37308 Heilbad Heiligenstadt (DE); BUSSE, Benedikt, 51491 Overath (DE); HELLMOLD, Jan-Hendrik, 37115 Duderstadt (DE)
(74) Vertreter: Gramm, Lins & Partner Patent- und Rechtsanwälte PartGmbB
(86) Internationale Anmeldenummer: PCT/EP2023/055464
(87) Internationale Veröffentlichungsnummer: WO 2023/166186

(56) Entgegenhaltungen:
- DE-A1- 102011 001 416
- DE-A1- 102014 013 716
- DE-A1- 102016 118 569
- US-A1- 2021 022 234

## Beschreibung

Die Erfindung betrifft eine Wundauflage zum Auflegen auf eine in einer Hautoberfläche befindlichen Wunde.

Zur Wundversorgung einer Wunde in einer Hautoberfläche ist es bekannt, eine Wundauflage auf die Wunde und die Hautoberfläche, in der sich die Wunde befindet, aufzulegen, um das Eindringen von Fremdkörpern in die Wunde zu verhindern und das Aufnehmen von Fluiden wie Blut und/oder Wundexsudat zu ermöglichen. Eine solche Wundauflage ist meist mehrschichtig bzw. mehrlagig aufgebaut und weist in der Regel ein saugfähiges Material auf, wie beispielsweise Zellstoff oder Baumwolle, auf und ist so zur Aufnahme und Speicherung von Blut und/oder Wundexsudat vorgesehen.

Aus der DE 20 2004 009 429 U1 ist ein absorbierender hygienischer Artikel mit einem absorbierenden Kern bekannt, der aus einem absorbierenden Faservlies besteht und auf wenigstens einer Seite mit einer fluidundurchlässigen Folie oder Vliesbahn abgedeckt ist. Dabei kann vorgesehen sein, dass dem Fasermaterial des Kerns superabsorbierende Polymere beigefügt sind.

Solche Superabsorber sind Materialien, die eine große Menge an Flüssigkeiten, wie beispielsweise Blut und/oder Wundexsudat, durch eine insbesondere chemisch-physikalische Bindung aufnehmen können. Die Aufnahmefähigkeit derartiger Superabsorber beträgt dabei meist ein Vielfaches des Eigengewichtes, wobei die Materialien dann in der Regel aufquellen und so die aufgenommenen Flüssigkeiten speichern und auch unter Druck nicht wieder abgeben. Durch die Verwendung derartiger Superabsorber als Materialien für die absorbiere Schicht einer Wundauflage lassen sich große Mengen an Blut und/oder Wundexsudat im Verhältnis zur Größe und Gewicht der Wundauflage aufnehmen, sodass eine optimale Wundversorgung bei einer möglichst langen Einsatzdauer sichergestellt werden kann.

Es ist auch bekannt, dass die Wundauflage mit einer selbstklebenden Schicht an der Wundseite der Wundauflage versehen ist, wie dies beispielsweise in der EP 2 338 449 A1 beschrieben ist. Hierdurch kann eine Fixierung der Wundauflage über der zu behandelnden Wunde auch über einen längeren Zeitraum sichergestellt werden.

Die DE 102017 100 192 A1 beschreibt eine permanente Wundauflage, bei der Wundsekret mittels einer Saugpumpe abgesaugt werden kann.

Es hat sich des Weiteren gezeigt, dass eine Plasmabehandlung für eine Wundheilung vorteilhaft sein kann. Dabei wird mithilfe einer eine Wundauflage bildenden Elektrodenanordnung bestehend aus einem Dielektrikum und einem in das Dielektrikum eingebetteten Elektrode eine dielektrisch behinderte Plasmaentladung zwischen einer flächigen Oberfläche der Elektrodenanordnung und eine als Gegenelektrode dienenden, zu behandelnden Oberfläche erzeugt, indem die Elektrode der Elektrodenanordnung mit einer elektrischen Wechselhochspannung gespeist wird.

Eine solche Elektrodenanordnung für eine dielektrisch behinderte Plasmaentladung ist beispielsweise aus der DE 10 2014 013 716 A1 bekannt. Die dort offenbarte Elektrodenanordnung weist dabei Durchgangsöffnungen im Dielektrikum auf, die mit Durchgangsöffnungen in der flächigen Elektrode fluchten, wobei die Durchgangsöffnungen der Elektrode größer sind als die Durchgangsöffnungen des Dielektrikums, sodass das Dielektrikum auch im Bereich der Durchgangsöffnungen die Elektrode vollständig abdeckt. Die in Richtung der Wunde zeigende Unterseite des Dielektrikums kann dabei mit einer Schicht bedeckt sein, bspw. aus einem Mull-Zellulosematerial oder einem pflegenden oder heilungsfördernden Material.

Eine ähnliche Elektrodenanordnung ist auch aus der DE 10 2016 118 569A1 bekannt, bei der die Elektrodenanordnung aus wenigstens zwei Teilelektroden besteht, die im Dielektrikum isoliert voneinander und nebeneinander eingebettet sind. Benachbarte Teilelektroden werden dabei von einer Steuereinrichtung mit bezüglich der Wellenform und der Spannungshöhe gegengleichen, sich kompensierenden Teil-Wechselspannungen gespeist.

Es ist Aufgabe der vorliegenden Erfindung eine verbesserte Wundauflage anzugeben, mit der die in der Hautoberfläche befindliche Wunde mit einem dielektrisch behinderten Plasma behandelt werden kann.

Die Aufgabe wird mit der Wundauflage gemäß Anspruch 1 erfindungsgemäß gelöst. Vorteilhafte Ausgestaltungen der Erfindung finden sich in den entsprechenden Unteransprüchen.

Gemäß Anspruch 1 wird eine Wundauflage zum Auflegen auf eine in einer Hautoberfläche befindlichen Wunde vorgeschlagen, wobei die Wundauflage eine Elektrodenanordnung zur Ausbildung einer dielektrisch behinderten Plasmaentladung durch mindestens eine mit einer Wechselhochspannung speisbare und in einem flächigen Dielektrikum vollständig eingebettete Elektrode aufweist. Das Dielektrikum bildet eine wundseitige und der zu behandelnden Hautoberfläche zugewandte Anlageseite. Die Elektrode weist über ihre Fläche verteilte Zwischenräume auf, die mit Durchgangsöffnungen des Dielektrikums fluchten, wobei die Durchgangsöffnungen des Dielektrikums kleinere Abmessungen aufweisen als die Zwischenräume der Elektrode, sodass das Dielektrikum auch im Bereich der Durchgangsöffnungen die Elektrode vollständig abdeckt, und sich von der wundseitigen Anlageseite bis zu einer der wundseitigen Anlageseite gegenüberliegenden Rückseite des Dielektrikums erstrecken und für ein Fluid durchlässig sind.

Erfindungsgemäß ist nunmehr vorgesehen, dass an der gegenüberliegenden Rückseite des Dielektrikums eine Fluidaufnahmelage zur Aufnahme eines durch die Durchgangsöffnungen des Dielektrikums von der wundseitigen Anlageseite zur Rückseite geleiteten Fluids anliegt, die einen Superabsorber aufweist oder aus einem solchen besteht. Die Durchgangsöffnungen im Dielektrikum sind dabei so ausgebildet, dass sich ein an der wundseitigen Anlageseite bildendes Fluid, wie bspw. Wundexsudat und/oder Blut, durch die Durchgangsöffnungen des Dielektrikums zu der Fluidaufnahmelage an der Rückseite der Elektrodenanordnung geleitet wird, so dass das Fluid durch den Superabsorber aufgenommen und gespeichert werden kann. Die Durchgangsöffnungen können dabei hinsichtlich ihrer Querschnittsgröße (Durchmesser) so ausgestaltet sein, dass das Aufsaugen des Fluids durch die Fluidaufnahmelage durch einen Kapillareffekt in den Durchgangsöffnungen unterstützt wird.

Hierdurch wird es möglich, eine Elektrodenanordnung zur Wundheilung, die beispielsweise in der DE 10 2014 013 716 A1 beschrieben, auch für Wunden mit einer starken Absonderung von Wundexsudat einzusetzen, da ein sich bildendes Wundexsudat durch die Elektrodenanordnung zu dem darüber liegenden Superabsorber abgeleitet wird. Darüber hinaus kann eine verlängerte Verweildauer der Wundauflage auf der zu behandelnden Hautoberfläche erreicht werden, da die Fluidaufnahmelage mit dem Superabsorber ein Vielfaches ihres Eigengewichtes an Fluid aufnehmen kann.

Der Superabsorber ist dabei so ausgebildet, dass die Wasseraufnahmefähigkeit des Superabsorbers einem Vielfachen des Eigengewichtes des Superabsorbers entspricht. So kann der Superabsorber eine Wasseraufnahmefähigkeit aufweisen, die wenigstens dem Dreifachen, vorzugsweise wenigstens dem Fünffachen seines Eigengewichtes entspricht.

Die Elektrode wird dabei auch im Bereich der Durchgangsöffnungen des Dielektrikums vollständig abgeschirmt, sodass sich ein an der Wunde gebildetes Wundexsudat sicher zum Superabsorber abführen lässt. Vorteilhafterweise führen die Zwischenräume in der Elektrode, die zumindest teilweise zur Bildung der Durchgangsöffnungen mit dem Dielektrikum besetzt sind, zu einem sicheren Halt der Elektrode innerhalb des Dielektrikums, was insbesondere dann vorteilhaft ist, wenn die Elektrode und das Dielektrikum flexibel ausgebildet sind und sich so an verschiedene Oberflächenformen der zu behandelnden Hautoberfläche anpassen müssen.

Die Elektrode kann flächig ausgebildet sein, wobei die Zwischenräume für die Durchgangsöffnungen im Dielektrikum durch Ausnehmungen in der flächigen Elektrode gebildet werden. Die Elektrode kann aber auch drahtförmig oder bandförmig ausgebildet sein und schneckenförmig oder mäanderförmig in dem Dielektrikum eingebettet sein. Zwischen dem drahtförmigen oder bandförmigen Verlauf der Elektrode werden Zwischenräume ausgebildet, die mit den Durchgangsöffnungen im Dielektrikum fluchten.

Die Elektrodenanordnung, genauer gesagt die Elektrode, kann dabei mit einem Hochspannungsgenerator verbunden werden oder sein, um die Elektrode mit einer Wechselhochspannung zu speisen. Der Hochspannungsgenerator kann dabei Teil einer Steuereinrichtung sein, die das Anlegen der Wechselhochspannung an der Elektrode mittels des Hochspannungsgenerators steuert. Der Hochspannungsgenerator und/oder die Steuereinrichtung können dabei außerhalb der Wundauflage bzw. außerhalb der Elektrodenanordnung vorgesehen sein.

In dieser Ausführungsform kann die Elektrodenanordnung ein Anschlussstück aufweisen, um die wenigstens eine Elektrode der Elektrodenanordnung mit der Hochspannungsstufe insbesondere bedarfsweise zu verbinden. Dabei wird eine elektrische Zuleitung von der Elektrode in das Anschlussstück geführt, wobei die Hochspannungsstufe dann mit der Zuleitung des Anschlussstückes elektrisch kontaktiert wird, bspw. mittels einer elektrischen Verbindungsanordnung.

Das Anschlussstück kann dabei einstückig mit dem Dielektrikum ausgebildet sein und insbesondere aus dem Material des Dielektrikums hergestellt sein. Das Anschlussstück isoliert dabei die Zuleitung der jeweiligen Elektrode bzw. Teilelektrode von der Umgebung. An einem der Elektrode gegenüberliegenden Ende der Zuleitung ist eine Verbindungsanordnung vorgesehen, um die Zuleitung in dem Anschlussstück mit dem Hochspannungsgenerator zu verbinden. In einer einfachen Ausführungsform ist hierfür eine Ausnehmung in dem Dielektrikum vorgesehen, welche das zu kontaktierende Ende der Zuleitung freilegt und so eine Kontaktierung mit dem Hochspannungsgenerator ermöglicht. Die Zuleitung wird dabei innerhalb der Isolierung kontaktiert, wobei die Verbindungsstelle nach außen durch die Anschlussanordnung isoliert wird, um einen sicheren Betrieb zu gewährleisten.

Das Anschlussstück enthält somit wenigstens einen elektrischen Leiter pro Elektrode bzw. pro Teilelektrode, um die jeweilige Elektrode bzw. Teilelektrode mit einer elektrischen Wechselspannung zu beaufschlagen.

Denkbar ist aber auch, dass der Hochspannungsgenerator und ggf. die Steuereinrichtung integraler Bestandteil der Elektrodenanordnung in Form eines Auflagestückes sind. Dabei werden der Hochspannungsgenerator und ggfs. die Steuereinrichtung bspw. in das Dielektrikum integriert, wobei der Hochspannungsgenerator dann über eine in dem Dielektrikum verlaufende elektrische Verbindung mit der Elektrode elektrisch kontaktiert wird.

In diesem Fall bedarf es keines separaten Anschlussstückes zur Verbindung der Elektrode mit einer Hochspannungsstufe eines Hochspannungsgenerators. Vielmehr werden alle für die Erzeugung eines dielektrisch behinderten Plasmas notwendigen Komponenten (insbesondere Hochspannungsstufe des Hochspannungsgenerators sowie gegebenenfalls Steuereinrichtung und ggfs. autarke elektrische Energiequelle) in das Auflagestück eingebettet, sodass ein autarker Betrieb der dielektrisch behinderten Plasmaerzeugung während der Wundbehandlung mit der Wundauflage möglich wird. Dabei kann vorgesehen sein, dass die autarke elektrische Energiequelle durch eine externe Energiequelle wiederaufladbar ist und hierfür an dem Anschlussstück ein insbesondere standardisierter Anschlussverbinder (beispielsweise ein USB-C Anschluss) vorgesehen ist.

Denkbar ist es schließlich auch, dass beide Alternativen miteinander kombiniert werden, um die größtmögliche Flexibilität zu erzeugen.

Die Elektrode der Elektrodenanordnung kann dabei vorzugsweise auch aus mehreren, voneinander isoliert im Dielektrikum eingebetteten Teilelektroden gebildet sein, sodass jede dieser Teilelektroden getrennt von den anderen jeweils mit einer Teil-Wechselhochspannung gespeist werden kann. Die Teilelektroden weisen dabei vorzugsweise zu einer der Hautoberfläche zugewandten Anlagefläche des Dielektrikums den gleichen Abstand auf.

Die Scheitelspannung der verwendeten Wechselhochspannung(en) kann zweckmäßigerweise zwischen ±1 kV und ±100 kV liegen. Die Wechselfrequenzen der Wechselhochspannungen liegen zweckmäßigerweise zwischen einigen 100 Hz und etwa 100 MHz. So kann die Pulsfrequenz, d.h. die Frequenz, mit der die einzelnen Impulse nacheinander ausgegeben werden, bei mehr als 90 Hz liegen, während die Impulsfrequenz selber bei mehr als 1 MHz liegt.

Gemäß einer Ausführungsform ist vorgesehen, dass die Elektrodenanordnung zur Ausbildung einer dielektrisch behinderten Plasmaentladung zwischen der mit einer Wechselhochspannung speisbaren Elektrode und der Hautoberfläche eines elektrisch leitfähigen Körpers, der als Masseelektrode dient, eingerichtet ist.

In diesem Fall ist die Hautoberfläche mit der zu behandelnden Wunde Teil eines elektrisch leitfähigen Körpers, der im Zusammenhang mit der Erzeugung des dielektrisch behinderten Plasmas die Masseelektrode bildet.

Denkbar ist aber auch, dass die Elektrodenanordnung eine von der Elektrode isolierte Gegenelektrode aufweist (bspw. ebenfalls in das Dielektrikum eingebettet), um eine dielektrisch behinderte Plasmaerzeugung zwischen der Elektrode und der Gegenelektrode der Elektrodenanordnung zu erreichen, wenn die Elektrode mit einer Wechselhochspannung gespeist und die Gegenelektrode bspw. auf einem Massepotential liegt.

Gemäß einer Ausführungsform ist vorgesehen, dass die Elektrodenanordnung mit der Elektrode und dem Dielektrikum flexibel ausgebildet sind. Hierdurch wird es möglich, dass die Wundauflage, die insgesamt flexibel ausgebildet ist, sich an jede Form der Hautoberfläche anpassen kann. Hierdurch wird eine intensivere und verbesserte Plasmaerzeugung erreicht.

Gemäß einer Ausführungsform ist vorgesehen, dass an der wundseitigen Anlageseite des Dielektrikums eine Wundauflageschicht anliegt, die in Richtung der Elektrodenanordnung für das durch die Fluidaufnahmelage aufzunehmende Fluid durchlässig ist. Die Wundauflageschicht kann bspw. aus einem Baumwollmaterial hergestellt sein und ermöglicht ein komfortables Tragen der Wundauflage auch über einen längeren Zeitraum hinweg. Die Wundauflageschicht kann dabei direkt auf der Wunde bzw. Hautoberfläche aufliegen, wobei das Dielektrikum dann direkt auf der Rückseite (von der Wunde abgewandte Seite) der Wundauflageschicht anliegt.

Gemäß einer Ausführungsform ist vorgesehen, dass die Elektrode der Elektrodenanordnung wenigstens zwei nebeneinander angeordneten und durch das Dielektrikum voneinander isolierten Teilelektroden aufweist und dass benachbarte Teilelektroden von einer Steuereinrichtung mit bezüglich der Wellenform und der Spannungshöhe gegengleichen, sich kompensierenden Teil-Wechselhochspannungen gespeist werden. Auch hierbei kann bevorzugt vorgesehen sein, dass die Hautoberfläche des elektrisch leitfähigen Körpers als Masseelektrode dient.

Die Teilelektroden liegen dabei in der flächigen Elektrodenanordnung nebeneinander in einer Ebene und weisen zu einer der Hautoberfläche zugewandten Anlagefläche des Dielektrikums den gleichen Abstand auf und sind durch das Dielektrikum voneinander isoliert. Durch die bezüglich der Wellenform und der Spannungshöhe gegengleichen, sich kompensierenden Teil-Wechselhochspannungen ergibt sich über die Periode der Wechselhochspannung ein sich nicht änderndes Mittenpotential, das dem Massepotential der Masseelektrode entspricht, wodurch Ansammlungen von Ladungsträgern auf der Haut des Patienten vermieden bzw. verringert werden. Außerdem kann mit einer geringeren Hochspannung gearbeitet werden.

Gemäß einer Ausführungsform ist vorgesehen, dass die Teilelektroden eine gleiche Größe aufweisen.

Gemäß einer Ausführungsform ist vorgesehen, dass die Durchgangsöffnungen des Dielektrikums einen Durchmesser zwischen 0,5 mm und 7 mm, vorzugsweise einen Durchmesser zwischen 0,75 mm und 5 mm aufweisen.

Gemäß einer Ausführungsform ist vorgesehen, dass die Fläche aller Durchgangsöffnungen des Dielektrikums einem Flächenanteil von 0,1% bis 60% der von der Elektrode durch ihre äußere Umrandung eingefassten Elektrodenfläche entspricht. Vorzugsweise entspricht der Flächenanteil 1% bis 35% der eingefassten Elektrodenfläche.

Gemäß einer Ausführungsform ist vorgesehen, dass die Wundauflage eine wundseitige Außenlage und eine gegenüberliegende rückseitige Außenlage hat, die in Bezug zur Hautoberfläche abgewandt ist, wobei zwischen der wundseitigen Außenlage und der rückseitigen Außenlage die Fluidaufnahmelage angeordnet ist, wobei zwischen der wundseitigen Außenlage und der Fluidaufnahmelage ein Aufnahmeraum gebildet wird, in der die Elektrodenanordnung angeordnet oder einsetzbar ist und wobei die wundseitige Außenlage in Richtung der Elektrodenanordnung für das durch die Fluidaufnahmelage aufzunehmende Fluid durchlässig ist.

In dieser Ausführungsform ist die wundseitige Außenlage, die als Wundauflageschicht mit der zu behandelnden Hautoberfläche und der Wunde in Berührung kommt, mit der rückseitigen Außenlage an zumindest einer Seite, vorzugsweise an zwei gegenüberliegenden Seiten, verbunden, so dass zwischen der darin eingeschlossenen Fluidaufnahmelage und der wundseitigen Außenlage ein Aufnahmeraum für die Elektrodenanordnung gebildet wird. Die Elektrodenanordnung kann bspw. bedarfsweise in den Aufnahmeraum eingesetzt werden und kann nach der Behandlung bzw. nach dem Entfernen der Wundauflage vom Patienten aus dem Aufnahmeraum entnommen und nach geeigneter Reinigung wiederverwendet werden.

Ist die Elektrodenanordnung in den Aufnahmeraum eingesetzt, so liegt die Elektrodenanordnung mit der wundseitigen Anlageseite an der Innenwandung der wundseitigen Außenlage einerseits und mit der gegenüberliegenden Rückseite des Dielektrikums an der Fluidaufnahmelage mit dem Superabsorber andererseits an. Das aufzunehmende Fluid wird dann durch die wundseitige Außenlage und durch die Durchgangsöffnungen des Dielektrikums der Elektrodenanordnung zu der Fluidaufnahmelage mit dem Superabsorber geleitet.

Es ist ebenfalls denkbar, dass die wundseitige Außenlage und die rückseitige Außenlage an drei Seiten miteinander verbunden sind, so dass ein taschenförmiger Aufnahmeraum gebildet ist.

Es ist ebenfalls denkbar, dass die wundseitige Außenlage und die rückseitige Außenlage allseitig miteinander verbunden sind, so dass die Elektrodenanordnung in dem Aufnahmeraum eingeschlossen ist. In dieser Ausführungsform sind lediglich die ggf. notwendigen elektrischen Anschlussverbindungen herausgeführt. Die Elektrodenanordnung ist hierbei in dem Aufnahmeraum eingeschlossen. Es kann vorgesehen sein, dass an einer Seite ein wiederverschließbares Element vorgesehen ist, beispielsweise ein Reißverschluss, um die Wundauflage bedarfsweise zu öffnen.

Gemäß einer Ausführungsform ist vorgesehen, dass aus dem Aufnahmeraum der Wundauflage ein Anschlussstück der Elektrodenanordnung herausragt, um die Elektrode mit einem Hochspannungsgenerator zum Speisen der Wechselhochspannung zu verbinden. Es kann sich dabei um ein Anschlussstück wie bereits vorstehend beschrieben handeln.

Gemäß einer Ausführungsform ist vorgesehen, dass die rückseitige Außenlage insbesondere flüssigkeitsundurchlässig ist. Die rückseitige Außenlage kann dabei nicht nur flüssigkeitsundurchlässig sein, sondern insbesondere auch atmungsaktiv und keimundurchlässig.

Gemäß einer Ausführungsform ist vorgesehen, dass die Anzahl der Durchgangsöffnungen des Dielektrikums mehr als 20, vorzugsweise mehr als 50, hierzu vorzugsweise mehr als 80, hierzu vorzugsweise mehr als 100 oder hierzu vorzugsweise mehr als 125 beträgt.

Es ist vorteilhaft, wenn die Durchgangsöffnungen im Dielektrikum derart ausgebildet sind, dass der Fluidtransort in Richtung Superabsorber durch einen Kapillareffekt unterstützt wird. Die Durchgangsöffnungen im Dielektrikum weisen dabei eine derartige Größe auf, dass im Verhältnis zur Dicke des Dielektrikums sich der Kapillareffekt derart positiv auswirkt, dass das abzuführende Fluid vorzugsweise bis zum Superabsorber transportiert wird.

Die Elektrodenanordnung kann in einem Gießverfahren, insbesondere einem Spritzgießverfahren, hergestellt sein. Bei dem Dielektrikum handelt es sich insbesondere um ein gießfähiges Material.

Die Erfindung wird anhand der beigefügten Figuren beispielhaft näher erläutert. Es zeigen:
- Figur 1: Generelle Darstellung der erfindungsgemäßen Wundauflage;
- Figur 2: Darstellung der Wundauflage mit Sicht auf die Elektrodenanordnung;
- Figur 3: Schnittdarstellung der Figur 1/2
- Figur 4: Vergrößerung der Schnittdarstellung aus Figur 3 im Abschnitt A.

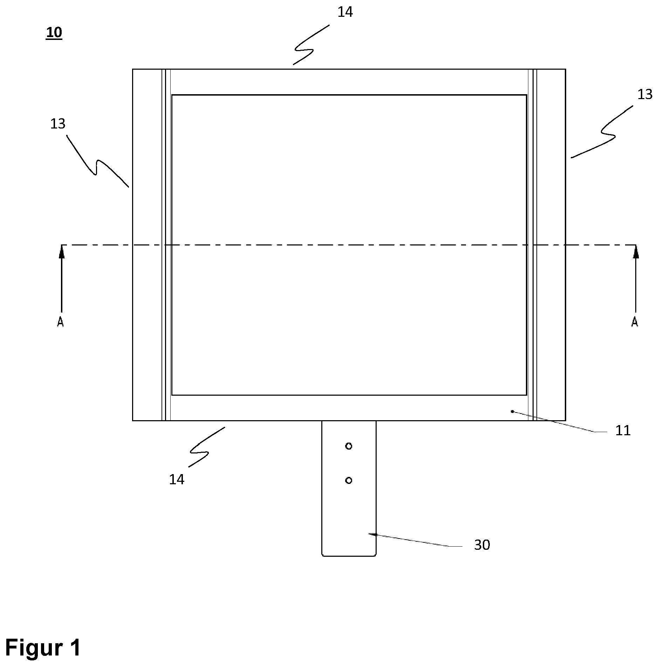

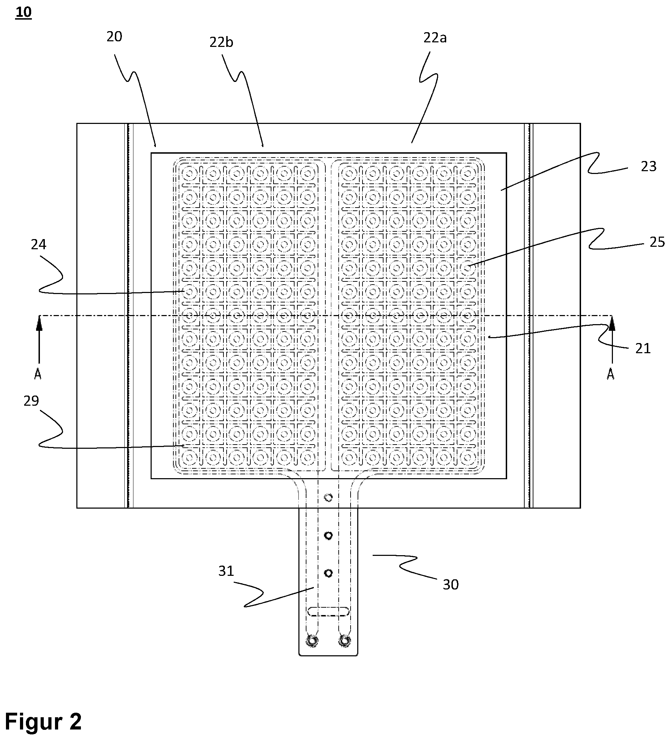

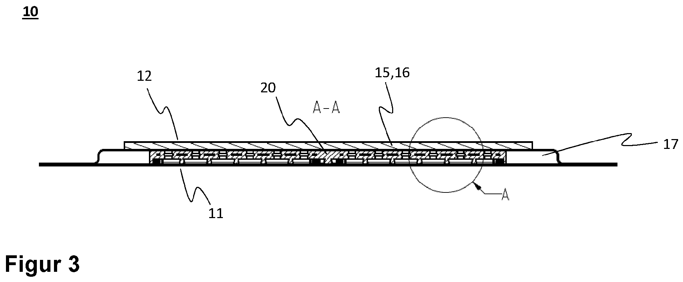

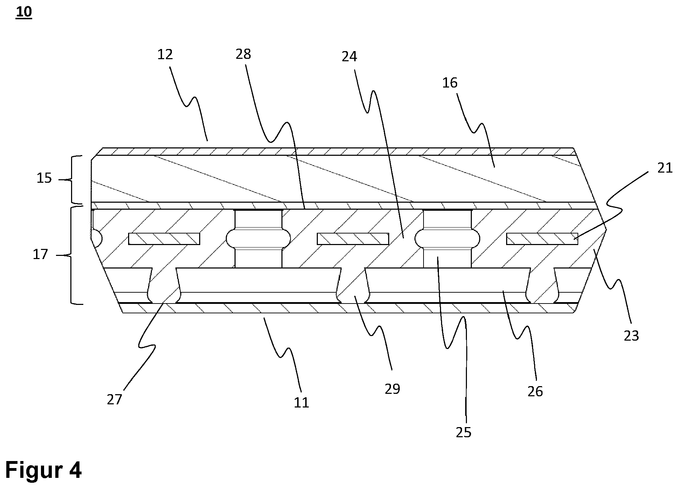

Die Figuren 1 bis 4 zeigen in jeweils verschiedenen Darstellungen und Perspektiven eine Wundauflage 10 in einer Ausführungsform. In den Figuren 1 und 2 ist dabei eine Sicht auf die Wundauflage 10 von der wundseitigen Außenlage 11 her gezeigt, während die rückseitige Außenlage 12 verdeckt ist. Die erfindungsgemäße Wundauflage 10 weist in der gezeigten Ausführungsform eine rechteckige Grundfläche auf, wobei in einem Randabschnitt 13 die wundseitige Außenlage 11 mit der rückseitigen Außenlage 12 fest verbunden ist. In einem Öffnungsabschnitt 14 ist die wundseitige Außenlage 11 mit der rückseitigen Außenlage 12 hingegen nicht fest verbunden, sodass sich eine Öffnung für einen Eingriff in die Wundauflage 10 bilden lässt.

Die wundseitige Außenlage 11 kann beispielsweise eine perforierte PE-Folie sein. Die wundseitige Außenlage 11 ist dabei insbesondere so ausgebildet, dass ein Fluid, beispielsweise ein Wundexsudat, welches sich zwischen der Wundauflage 10 und der zu behandelnden Wunde bilden kann, in das Innere der Wundauflage 10 gelangt. Die rückseitige Außenlage 12 kann dabei insbesondere flüssigkeitsundurchlässig ausgebildet sein. Zwischen der wundseitigen Außenlage 11 und der rückseitigen Außenlage 12 befindet sich eine Fluidaufnahmelage 15, die einen Superabsorber 16 aufweist.

Zwischen der Fluidaufnahmelage 15 mit dem Superabsorber 16 und der wundseitigen Außenlage 11 wird ein Aufnahmeraum 17 gebildet, in den eine Elektrodenanordnung 20 eingesetzt ist. Der Öffnungsabschnitt 14 erlaubt dabei einen Eingriff in den Aufnahmeraum 17, so dass die Elektrodenanordnung 20 in den Aufnahmeraum 17 eingesetzt werden kann. Nach Beendigung der Plasmabehandlung kann die Elektrodenanordnung 20 wieder entnommen, gereinigt und wiederverwendet werden, während die übrigen Bestandteile der Wundauflage 10 fachgerecht entsorgt werden können.

Die Elektrodenanordnung 20 hat im Ausführungsbeispiel der Figuren 1 bis 4 eine Elektrode 21 mit zwei Teilelektroden 22a, 22b, die in einem Dielektrikum 23 fest eingebettet sind.

Die Teilelektroden 22a, 22b sind dabei flächig ausgebildet und weisen Ausnehmungen bzw. Öffnungen 24 auf, die dafür vorgesehen sind, mit Durchgangsöffnungen 25 in dem Dielektrikum 23 zu fluchten. Die Durchgangsöffnungen 25 in dem Dielektrikum sind dabei kleiner als die Ausnehmungen 24 in den Teilelektroden 22a, 22b, sodass auch im Bereich der Durchgangsöffnungen 25 in dem Dielektrikum die Elektroden 22a, 22b vollständig abgeschirmt werden.

Wird eine solche Elektrodenanordnung 20 nun in den in der Wundauflage 10 gebildeten Aufnahmeraum 17 eingesetzt, so bilden die Durchgangsöffnungen 25 in dem Dielektrikum einen Fluidkanal von der wundseitigen Außenlage 11 zu der Fluidaufnahmelage 15 mit dem Superabsorber 16. Die Elektrodenanordnung 20 liegt dann mit einer der wundseitigen Außenlage 11 zugewandten Anlageseite 27 des Dielektrikums 23 an der Innenwandung der wundseitigen Außenlage 11 einerseits und mit einer abgewandten Rückseite 28 des Dielektrikums 23 an der Fluidaufnahmelage 15 mit dem Super-absorber 16 andererseits an, sodass sich an der wundseitigen Außenlage 11 bildendes Wundexsudat und/oder Blut dann durch die Elektrodenanordnung 20 bis zu dem Superabsorber 16 geleitet werden kann.

An der Anlageseite 27 des Dielektrikums 23 befinden sich ein durch das Dielektrikum 23 gebildetes Abstandsgitter 29, welches längs und quer dazu verlaufende dünne Stege aufweist. Durch das Abstandsgitter 29 werden Vorkammern 26 für einen Teil oder alle Durchgangsöffnungen 25 gebildet, in denen sich das dielektrisch behinderte Plasma bilden und ausbreiten kann, wenn die Elektrode 21 mit einer Wechselhochspannung gespeist wird. Bildet sich Wundexsudat und/oder Blut im Bereich der Wunde, so können vereinzelt die Vorkammern 26 gefüllt und das Wundexsudat und/oder Blut in Richtung Superabsorber unter Ausnutzung des Kapillareffektes in den Durchgangsöffnungen 25 abgeleitet werden.

Zur Herstellung der Elektrodenanordnung kann zunächst eine erste Dielektrikumsschicht durch ein gießfähiges Material in einer Form gebildet werden, in die die Elektrode 21 aufgelegt wird. Anschließend wird durch eine darauf aufgebrachte zweite Dielektrikumsschicht aus dem gießfähigen Material die Elektrode allseits durch das Dielektrikum abgeschirmt. Dabei werden zur Bildung der Durchgangsöffnungen 25 Kerne eingesetzt, die nach der Herstellung der Elektrodenanordnung entfernt werden.

Das Dielektrikum 23 formt an einer Seite ein Anschlussstück 30 aus, in das sich für jede Teilelektrode 22a, 22b getrennt eine jeweilige elektrische Zuleitung 31 hinein erstreckt. Das Anschlussstück 30 ragt dabei aus der äußeren Umfassung der Wundauflage 10, insbesondere im Bereich des Öffnungsabschnittes 14, hinaus und ermöglicht so die elektrische Kontaktierung der Elektrode 21 mit einem Wechselhochspannungsgenerator. **In** einem Endabschnitt der Zuleitungen 31 ist dabei eine Ausnehmung in der Isolierung vorgesehen, um so die Zuleitungen 31 von außen zugänglich zu machen. Durch eine geeignete Anschlussanordnung kann dann eine elektrische Verbindung mit der Zuleitung 31 hergestellt werden, indem die Anschlussanordnung in die Ausnehmung der Isolierung eingreift und die Zuleitung innerhalb der Isolierung kontaktiert, wobei die Verbindungsstelle nach außen durch die Anschlussanordnung isoliert wird.

Die rückseitige Außenlage 12 ist dabei so ausgebildet, dass die vor der Fluidaufnahme eher pulverförmig gepresste Fluidaufnahmelage 15 mit dem Superabsorber 16 in ihrer Form gehalten wird.

### Bezugszeichenliste

- 10: Wundauflage
- 11: wundseitige Außenlage
- 12: rückseitige Außenlage
- 13: Randabschnitt
- 14: Öffnungsabschnitt
- 15: Fluidaufnahmelage
- 16: Superabsorber
- 17: Aufnahmeraum
- 20: Elektrodenanordnung
- 21: Elektrode
- 22a,22b: Teilelektroden
- 23: Dielektrikum
- 24: Ausnehmungen/Zwischenräume in der Elektrode
- 25: Durchgangsöffnungen im Dielektrikum
- 26: Vorkammern
- 27: Anlageseite des Dielektrikums
- 28: Rückseite des Dielektrikums
- 29: Abstandsgitter
- 30: Anschlussstück
- 31: Zuleitung im Anschlussstück

## Patentansprüche

1. Wundauflage (10) zum Auflegen auf eine in einer Hautoberfläche befindlichen Wunde mit
einer Elektrodenanordnung (20) zur Ausbildung einer dielektrisch behinderten Plasmaentladung durch mindestens eine mit einer Wechselhochspannung speisbaren und in einem flächigen Dielektrikum (23) vollständig eingebetteten Elektrode (21, 22a, 22b),
wobei das Dielektrikum (23) eine wundseitige Anlageseite (27) bildet und die Elektrode (21) über ihre Fläche verteilte Zwischenräume (24) aufweist, die mit Durchgangsöffnungen (25) des Dielektrikums (23) fluchten,
wobei die Durchgangsöffnungen (25) des Dielektrikums kleinere Abmessungen aufweisen als die Zwischenräume (24) der Elektrode (21), sodass das Dielektrikum (23) auch im Bereich der Durchgangsöffnungen (25) die Elektrode (21) vollständig abdeckt, und sich von der wundseitigen Anlageseite (27) bis zu einer der wundseitigen Anlageseite (27) gegenüberliegenden Rückseite (28) des Dielektrikums (23) erstrecken und für ein Fluid durchlässig sind,
**dadurch gekennzeichnet, dass**
an der gegenüberliegenden Rückseite (28) des Dielektrikums (23) eine Fluidaufnahmelage (15) zur Aufnahme eines durch die Durchgangsöffnungen (25) des Dielektrikums (23) von der wundseitigen Anlageseite (27) zur Rückseite (28) geleiteten Fluids anliegt, die einen Superabsorber (16) aufweist oder aus einem solchen besteht.

2. Wundauflage (10) nach Anspruch 1, **dadurch gekennzeichnet, dass**
die Durchgangsöffnungen hinsichtlich ihrer Querschnittsgröße so ausgestaltet sind, dass das Aufsaugen des Fluids durch die Fluidaufnahmelage durch einen Kapillareffekt in den Durchgangsöffnungen unterstützt wird.

3. Wundauflage (10) nach einem der vorhergehenden Ansprüche, **dadurch gekennzeichnet, dass**
an der wundseitigen Anlageseite (27) des Dielektrikums (23) eine Wundauflageschicht anliegt, die in Richtung der Elektrodenanordnung (20) für das durch die Fluidaufnahmelage (15) aufzunehmende Fluid durchlässig ausgebildet ist.

4. Wundauflage (10) nach einem der vorhergehenden Ansprüche, **dadurch gekennzeichnet, dass**
die Durchgangsöffnungen (25) des Dielektrikums einen Durchmesser zwischen 0,5 mm und 7 mm, vorzugsweise einen Durchmesser zwischen 0,75 mm und 5 mm aufweisen.

5. Wundauflage (10) nach einem der vorhergehenden Ansprüche, **dadurch gekennzeichnet, dass**
die Fläche aller Durchgangsöffnungen (25) des Dielektrikums (23) einem Flächenanteil von 0,1% bis 60% der von der Elektrode (21) durch ihre äußere Umrandung eingefasste Elektrodenfläche entspricht.

6. Wundauflage (10) nach einem der vorhergehenden Ansprüche, **dadurch gekennzeichnet, dass**
die Wundauflage (10) eine wundseitige Außenlage (11) und eine gegenüberliegende rückseitige Außenlage (12) hat, die in Bezug zur Hautoberfläche abgewandt ist,
wobei zwischen der wundseitigen Außenlage (11) und der rückseitigen Außenlage (12) die Fluidaufnahmelage (15) angeordnet ist,
wobei zwischen der wundseitigen Außenlage (11) und der Fluidaufnahmelage (15) ein Aufnahmeraum (17) gebildet wird, in dem die Elektrodenanordnung (20) angeordnet oder einsetzbar ist und
wobei die wundseitige Außenlage (11) in Richtung der Elektrodenanordnung (20) für das durch die Fluidaufnahmelage (15) aufzunehmende Fluid durchlässig ist.

7. Wundauflage (10) nach Anspruch 6, **dadurch gekennzeichnet, dass**
aus dem Aufnahmeraum (17) der Wundauflage (10) ein Anschlussstück (30) der Elektrodenanordnung (20) herausragt, um die Elektrode (21) mit einem Hochspannungsgenerator zum Speisen einer Wechselhochspannung zu verbinden.

8. Wundauflage (10) nach Anspruch 6 oder 7, **dadurch gekennzeichnet, dass**
die wundseitige Außenlage und die rückseitige Außenlage an drei Seiten miteinander verbunden sind, so dass ein taschenförmiger Aufnahmeraum gebildet ist.

9. Wundauflage (10) nach einem der vorhergehenden Ansprüche, **dadurch gekennzeichnet, dass** die Anzahl der Durchgangsöffnungen des Dielektrikums mehr als 20, vorzugsweise mehr als 50, hierzu vorzugsweise mehr als 80, hierzu vorzugsweise mehr als 100 oder hierzu vorzugsweise mehr als 125 beträgt.

10. Wundauflage (10) nach einem der vorhergehenden Ansprüche, **dadurch gekennzeichnet, dass** die Wasseraufnahmefähigkeit des Superabsorbers (16) einem Vielfachen des Eigengewichtes des Superabsorbers (16) entspricht.

11. Wundauflage (10) nach einem der vorhergehenden Ansprüche, **dadurch gekennzeichnet, dass**
die Elektrodenanordnung (20) zur Ausbildung einer dielektrisch behinderten Plasmaentladung zwischen der mit einer Wechselhochspannung speisbaren Elektrode (21) und der Hautoberfläche eines elektrisch leitfähigen Körpers, der als Masseelektrode dient, eingerichtet ist.

12. Wundauflage (10) nach einem der vorhergehenden Ansprüche, **dadurch gekennzeichnet, dass**
die Elektrodenanordnung (20) mit der Elektrode (21) und dem Dielektrikum (23) flexibel ausgebildet sind.

13. Wundauflage (10) nach einem der vorhergehenden Ansprüche, **dadurch gekennzeichnet, dass**
die Elektrode (21) der Elektrodenanordnung (20) wenigstens zwei nebeneinander angeordnete und durch das Dielektrikum (23) voneinander isolierten Teilelektroden (22a,22b) aufweist und dass benachbarte Teilelektroden (22a,22b) von einer Steuereinrichtung mit bezüglich der Wellenform und der Spannungshöhe gegengleichen, sich kompensierenden Teil-Wechselhochspannungen gespeist werden.

14. Wundauflage (10) nach Anspruch 13, **dadurch gekennzeichnet, dass** die Teilelektroden (22a, 22b) eine gleiche Größe aufweisen.

## Claims

1. A wound dressing (10) for placement on a wound located on a skin surface with an electrode arrangement (20) for forming a dielectric barrier plasma discharge by way of at least one electrode (21, 22a, 22b) that can be supplied with a high AC voltage and is completely embedded in a flat dielectric (23),
wherein the dielectric (23) forms a wound-side application side (27) and the electrode (21) has intermediate spaces (24) dispersed across its surface that are aligned with passage openings (25) of the dielectric (23),
the passage openings (25) of the dielectric having smaller dimensions than the intermediate spaces (24) of the electrode (21), so that the dielectric (23) also completely covers the electrode (21) in the area of the passage openings (25) and extends from the wound-side application side (27) to a rear side (28) of the dielectric (23) opposite the wound-side application side (27) and is permeable to a fluid,
**characterised in that**
a fluid absorption layer (15) for absorbing a fluid conducted through the passage openings (25) of the dielectric (23) from the wound-side application side (27) to the rear side (28), which has a super-absorber (16) or consists of such a super-absorber, rests against the opposite rear-side (28) of the dielectric (23).

2. The wound dressing (10) according to claim 1, **characterised in that**
the cross-sectional size of the passage openings is designed in such a way that the absorption of the fluid by the fluid absorption layer is supported by a capillary effect in the passage openings.

3. The wound dressing (10) according to one of the preceding claims, **characterised in that**
a wound contact layer rests against the wound-side application side (27) of the dielectric (23), the wound contact layer being designed to be permeable to the fluid to be absorbed by the fluid absorption layer (15) in the direction of the electrode arrangement (20).

4. The wound dressing (10) according to one of the preceding claims, **characterised in that**
the passage openings (25) of the dielectric have a diameter of between 0.5mm and 7mm, preferably a diameter of between 0.75mm and 5mm.

5. The wound dressing (10) according to one of the preceding claims, **characterised in that**
the area of all passage openings (25) of the dielectric (23) corresponds to an area of 0.1% to 60% of the electrode area enclosed by the outer edge of the electrode (21).

6. The wound dressing (10) according to one of the preceding claims, **characterised in that**
the wound dressing (10) has a wound-side outer layer (11) and an opposite rear-side outer layer (12) that faces away in relation to the skin surface.
wherein the fluid absorption layer (15) is arranged between the wound-side outer layer (11) and the rear-side outer layer (12),
wherein an accommodation space (17) is formed between the wound-side outer layer (11) and the fluid absorption layer (15) in which the electrode arrangement (20) is arranged or can be inserted, and
wherein the wound-side outer layer (11) is permeable to the fluid to be absorbed by the fluid absorption layer (15) in the direction of the electrode arrangement (20).

7. The wound dressing (10) according to claim 6, **characterised in that**
a connecting piece (30) of the electrode arrangement (20) protrudes from the accommodation space (17) of the wound dressing (10) in order to connect the electrode (21) to a high-voltage generator for supplying a high AC voltage.

8. The wound dressing (10) according to claim 6 or 7, **characterised in that**
the wound-side outer layer and the rear-side outer layer are connected to each other on three sides, thereby forming a pocket-like accommodation space.

9. The wound dressing (10) according to one of the preceding claims, **characterised in that** there are more than 20 passage openings of the dielectric, preferably more than 50, preferably more than 80, preferably more than 100 or preferably more than 125.

10. The wound dressing (10) according to one of the preceding claims, **characterised in that** the water absorption capacity of the super-absorber (16) corresponds to the super-absorber's (16) own weight many times over.

11. The wound dressing (10) according to one of the preceding claims, **characterised in that**
the electrode arrangement (20) is configured between the electrode (21) that can be supplied with a high AC voltage and the skin surface of an electrically conductive body, which serves as a ground electrode, in order to form a dielectric barrier plasma discharge.

12. The wound dressing (10) according to one of the preceding claims, **characterised in that**
the electrode arrangement (20) with the electrode (21) and the dielectric (23) are designed to be flexible.

13. The wound dressing (10) according to one of the preceding claims, **characterised in that**
the electrode (21) of the electrode arrangement (20) has at least two partial electrodes (22a, 22b) that are arranged next to each other and insulated against each other by the dielectric (23), and that neighbouring partial electrodes (22a, 22b) are supplied by a control device with compensating partial alternating voltages of opposite waveforms and voltage levels.

14. The wound dressing (10) according to claim 13, **characterised in that** the partial electrodes (22a, 22b) are the same size.

## Revendications

1. Pansement (10) destiné à être appliqué sur une plaie située à la surface de la peau, comprenant
un ensemble d'électrode (20) pour former une décharge de plasma à barrière diélectrique à l'aide d'au moins une électrode (21, 22a, 22b) susceptible d'être alimentée en une haute tension alternative et noyée complètement dans un diélectrique plat (23),
dans lequel le diélectrique (23) forme une face d'appui (27) côté plaie, et l'électrode (21) présente des espaces intermédiaires (24) répartis sur sa surface, qui sont alignés avec des ouvertures de passage (25) du diélectrique (23),
dans lequel les ouvertures de passage (25) du diélectrique présentent des dimensions plus petites que les espaces intermédiaires (24) de l'électrode (21), de sorte que le diélectrique (23) recouvre complètement l'électrode (21) également dans la zone des ouvertures de passage (25), et s'étendent depuis la face d'appui (27) côté plaie jusqu'à une face arrière (28) du diélectrique (23) opposée à la face d'appui (27) côté plaie et sont perméables à un fluide,
**caractérisé en ce que**
une couche d'absorption de fluide (15) est prévue sur la face arrière opposée (28) du diélectrique (23), qui est destinée à absorber un fluide, acheminé à travers les ouvertures de passage (25) du diélectrique (23) depuis la face d'appui (27) côté plaie vers la face arrière (28), et qui comprend un superabsorbant (16) ou est constituée d'un tel superabsorbant.

2. Pansement (10) selon la revendication 1, **caractérisé en ce que**
les ouvertures de passage sont conçues, en termes de taille de section transversale, de manière à favoriser l'absorption du fluide par la couche d'absorption de fluide grâce à un effet capillaire dans les ouvertures de passage.

3. Pansement (10) selon l'une des revendications précédentes, **caractérisé en ce que**
une couche de pansement s'appuie contre la face d'appui (27) côté plaie du diélectrique (23), laquelle couche est conçue de manière perméable au fluide à absorber par la couche d'absorption de fluide (15), en direction de l'ensemble d'électrode (20).

4. Pansement (10) selon l'une des revendications précédentes, **caractérisé en ce que**
les ouvertures de passage (25) du diélectrique ont un diamètre compris entre 0,5 mm et 7 mm, de préférence un diamètre compris entre 0,75 mm et 5 mm.

5. Pansement (10) selon l'une des revendications précédentes, **caractérisé en ce que**
la surface de toutes les ouvertures de passage (25) du diélectrique (23) correspond à une proportion de surface comprise entre 0,1 % et 60 % de la surface d'électrode délimitée par le bord extérieur de l'électrode (21).

6. Pansement (10) selon l'une des revendications précédentes, **caractérisé en ce que**
le pansement (10) comprend une couche extérieure (11) côté plaie et une couche extérieure opposée (12) côté arrière qui est détournée de la surface de la peau,
la couche d'absorption de fluide (15) étant disposée entre la couche extérieure (11) côté plaie et la couche extérieure (12) côté arrière,
un espace de réception (17) étant formé entre la couche extérieure (11) côté plaie et la couche d'absorption de fluide (15), espace dans lequel l'ensemble d'électrode (20) est disposé ou peut être inséré, et
la couche extérieure (11) côté plaie est perméable au fluide à absorber par la couche d'absorption de fluide (15), dans la direction de l'ensemble d'électrode (20).

7. Pansement (10) selon la revendication 6, **caractérisé en ce que**
une pièce de raccordement (30) de l'ensemble d'électrode (20) dépasse de l'espace de réception (17) du pansement (10) afin de relier l'électrode (21) à un générateur haute tension destiné à fournir une haute tension alternative.

8. Pansement (10) selon la revendication 6 ou 7, **caractérisé en ce que**
la couche extérieure côté plaie et la couche extérieure côté arrière sont reliées entre elles sur trois côtés, de manière à former un espace de réception en forme de poche.

9. Pansement (10) selon l'une des revendications précédentes, **caractérisé en ce que**
le nombre d'ouvertures de passage du diélectrique est supérieur à 20, de préférence supérieur à 50, de préférence supérieur à 80, de préférence supérieur à 100 ou de préférence supérieur à 125.

10. Pansement (10) selon l'une des revendications précédentes, **caractérisé en ce que**
la capacité d'absorption d'eau du superabsorbant (16) correspond à un multiple du poids propre du superabsorbant (16).

11. Pansement (10) selon l'une des revendications précédentes, **caractérisé en ce que**
l'ensemble d'électrode (20) est conçu pour former une décharge de plasma à barrière diélectrique entre l'électrode (21), susceptible d'être alimentée en haute tension alternative, et la surface cutanée d'un corps électriquement conducteur servant d'électrode de masse.

12. Pansement (10) selon l'une des revendications précédentes, **caractérisé en ce que**
l'ensemble d'électrode (20) munie de l'électrode (21) et du diélectrique (23) est réalisé de manière flexible.

13. Pansement (10) selon l'une des revendications précédentes, **caractérisé en ce que**
l'électrode (21) de l'ensemble d'électrode (20) comprend au moins deux électrodes partielles (22a, 22b) disposées l'une à côté de l'autre et isolées l'une de l'autre par le diélectrique (23), et
**en ce que** les électrodes partielles (22a, 22b) voisines sont alimentées en hautes tensions alternatives partielles compensatrices, opposées en termes de forme d'onde et de niveau de tension, au moyen d'un dispositif de commande.

14. Pansement (10) selon la revendication 13, **caractérisé en ce que** les électrodes partielles (22a, 22b) ont la même taille.
